# EUROPÄISCHE PATENTSCHRIFT

(11) **EP 0 885 185 B1**
(45) Veröffentlichungstag und Bekanntmachung des Hinweises auf die Patenterteilung: **02.05.2001**
(21) Anmeldenummer: 97900578.2
(22) Anmeldetag: 09.01.1997
(51) Int. Cl.: C07C 229/26, C11D 1/88, C11D 1/90, C07C 229/16

(54) **BETAIN-GEMINITENSIDE AUF DER BASIS VON AMINEN**
BETAINE GEMINI SURFACTANTS MADE FROM AMINES
TENSIOACTIFS JUMELES BETAINIQUES A BASE D'AMINES

(30) Priorität: 02.03.1996 DE 19608117
(43) Veröffentlichungstag der Anmeldung: 23.12.1998
(73) Patentinhaber: RWE-DEA Aktiengesellschaft für Mineraloel und Chemie, 22297 Hamburg (DE)
(72) Erfinder: KWETKAT, Klaus, D-44534 Lünen (DE)
(74) Vertreter: Schupfner, Gerhard D., Dr. Dipl.-Chem.
(86) Internationale Anmeldenummer: EP9700055
(87) Internationale Veröffentlichungsnummer: WO9731890

(56) Entgegenhaltungen:
- EP-A- 0 623 587
- EP-A- 0 708 079
- DE-C- 902 258
- GB-A- 1 137 491
- PATENT ABSTRACTS OF JAPAN vol. 096, no. 007, 31.Juli 1996 & JP 08 081354 A (KAO CORP), 26.März 1996,
- PATENT ABSTRACTS OF JAPAN vol. 096, no. 007, 31.Juli 1996 & JP 08 060183 A (KAO CORP), 5.März 1996,

## Beschreibung

Die Erfindung betrifft amphotere Tenside mit mindestens zwei hydrophilen und mindestens zwei hydrophoben Gruppen (Betain-Geminitenside), die so aufgebaut sind, daß jeweils komplette Tensideinheiten miteinander verknüpft werden.

Als ämphiphile Substanzen sind eine große Vielfalt an anionischen, kationischen, nichtionischen und zwitterionischen Verbindungen bekannt. Die weitaus meisten dieser Substanzen bestehen aus einer hydrophilen Kopfgruppe und wenigstens einem hydrophoben Teil. Aus ökologischen Gründen z. B. bezüglich der Verringerung des Verpackungs- und Transportaufwandes sowie hinsichtlich einer sparsamen Rohstoffnutzung gibt es die Notwendigkeit, immer größere Wirkung pro Masse an eingesetzter Substanz zu erzielen. Da eine Optimierung durch Mischung von amphiphilen Substanzen nur begrenzt weiterführt, sind neue amphiphile Substanzen mit einem höheren Wirkungsgrad erforderlich. Es müssen daher insbesondere Stoffe mit niedrigeren kritischen Mizellbildungskonzentrationen und niedrigeren Grenzflächenspannungen gefunden werden, um die Einsatzmengen an Wirksubstanz deutlich reduzieren zu können. Zudem müssen sie leicht, am besten aus leicht zugänglichen Ausgangssubstanzen, erhältlich sein.

Erste Lösungsansätze in Richtung auf leistungsfähigere amphiphile Substanzen durch Verdoppelung eines Teils der Struktur (hydrophile Kopfgruppe, hydrophobe Gruppe) sind bereits bekannt. So können kationische grenzflächenaktive Verbindungen durch die Addition von langkettigen Alkylhalogeniden an permethylierte Alkylendiamine erhalten werden [R. Zana, M. Benrraou, R. Rueff, Langmuir, 7 (1991) 1072; R. Zana, Y. Talmon, Nature, 362 (1993) 228; E. Alami, G. Beinert, P. Marie, R. Zana, Langmuir, 9 (1993) 1465]. Offenbar ist eine Leistungssteigerung bei grenzflächenaktiven Verbindungen durch die geeignete Verknüpfung mehrerer Tensideinheiten (aus hydrophiler Kopfgruppe und hydrophober Kette) möglich [R. Zana, H. Levy, D. Papoutsi, G. Beinert, Langmuir, 11 (1995) 3694.].

In der EP 0 708 079-A1 werden 2-Hydroxypropandiamin - Derivate und eine Tensid-Zusammensetzung enthaltend diese, offenbart, wobei die 2-Hydroxypropandiamin-Derivate folgende Struktur aufweisen: 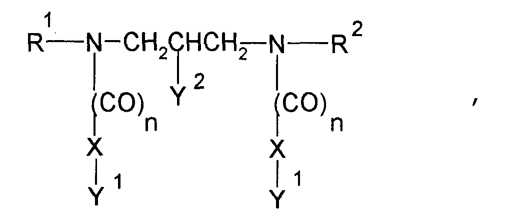 worin
- R¹ und R²: unabhängig voneinander für eine lineare oder verzweigte Alkyl- oder Alkylengruppe mit 6 bis 36 Kohlenstoffatomen,
- X: für eine C1- bis C6- Alkyl- oder Alkylen-Gruppe steht , die ggf. mit einer Hydroxyl-Gruppe (-OH), einer Sulfonsäure-Gruppe (-SO₃H) oder einer Carboxyl-Gruppe (-COOH) substituiert sein kann,
- Y¹: eine Sulfonsäure-Gruppe (-SO₃H), eine Schwefelsäure-Gruppe oder eine Carboxyl-Gruppe (-COOH) ist,
- Y²: eine Hydroxyl-Gruppe (-OH), einen Schwefelsäure-Rest (-OSO₃H) oder

-O-C(=O)X-COOH

darstellt, X die Bedeutung von oben hat und
- n: 0 oder 1 bedeutet.

Die EP 0 708 079-A1 offenbart auch die Salze der oben wiedergegeben Säuren sowie entsprechende quaternisierte Derivate. Die EP 0 708 079-A1 ist ein "Artikel 54(3)-Dokument" mit Wirkung für die Vertragsstaaten Deutschland (DE), Spanien (ES), Frankreich (FR) und Großbritannien (GB).

Da amphotere Verbindungen als besonders hautmild bekannt sind, bestand somit insbesondere die Aufgabe, amphotere grenzflächenaktive Verbindungen herzustellen, die wenigstens zwei hydrophile und wenigstens zwei hydrophobe Gruppen aufweisen, wobei die amphiphilen Verbindungen einen sehr hohen Wirkungsgrad, bezogen auf die Einsatzmenge, haben, und die darüber hinaus aus technisch leicht verfügbaren Rohstoffen ohne unerwünschte Nebenprodukte hergestellt werden können. In der Offenlegungsschrift DE 43 09 900 sind amphotere Tenside beschrieben, die zwei oder mehr hydrophile Gruppen enthalten können, doch führt die Art der Verknüpfung der dort beschriebenen Struktureinheiten nicht zu einer Leistungssteigerung im Vergleich zu monomeren amphoteren Tensiden.

Die Aufgabe der Leistungssteigerung wird erfindungsgemäß durch amphotere Verbindungen mit mindestens zwei hydrophilen und mindestens zwei hydrophoben Gruppen gelöst, die so aufgebaut sind, daß jeweils komplette Tensideinheiten miteinander verknüpft sind. Gegenstand der Erfindung sind also Verbindungen gemäß der Formel (I):

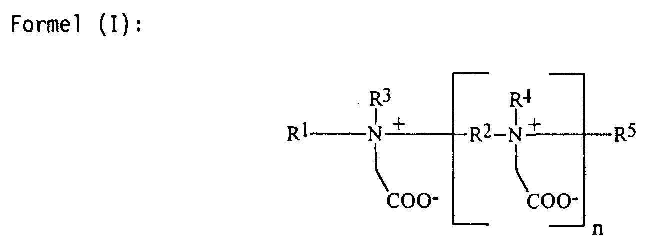

Hierbei steht R¹ für einen gesättigten oder ungesättigten, verzweigten oder unverzweigten, cyclischen oder acyclischen Kohlenwasserstoffrest mit 6 bis 22 Kohlenstoffatomen, R³ für ein Wasserstoffatom oder einen Alkylrest mit 1 bis 4 Kohlenstoffatomen, R⁴ unabhängig von den anderen Substituenten für einen gesättigten oder ungesättigten, verzweigten oder unverzweigten, cyclischen oder acyclischen Kohlenwasserstoffrest mit 6 bis 22 Kohlenstoffatomen und R⁵ für ein Wasserstoffatom oder einen Alkylrest mit 1 bis 4 Kohlenstoffatomen. n kann Werte von 1 bis 100 000 annehmen, bevorzugt von 1 bis 1 000, besonders bevorzugt von 1 bis 100 und ganz besonders bevorzugt von 1 bis 10, wobei auch Gemische verschiedener Homologer auftreten können. Deshalb kann n im Mittel auch gebrochene Werte annehmen.

Der Spacer R² ist ausgewählt aus der Gruppe:
-(CH₂)₂-, -(CH₂)₃-, -(CH₂)₄-, -(CH₂)₅-,
-(CH₂)₆-, -CH₂-CH(CH₃)-(CH₂)₃ -, weiterhin -(CH₂)₂-O-(CH₂)₂-,
-(CH₂)₂-O-(CH₂)₃-, -(CH₂)₃-O-(CH₂)₃-, -(CH₂)₂-O-(CH₂)₂-O-(CH₂)₂-,
-(CH₂)₃-O-(CH₂)₂-O-(CH₂)₃-, -(CH₂)₂-O-(CH₂)₃-O-(CH₂)₂-, -(CH₂)₂-NH-(CH₂)₃- und
-(CH₂)₂-NH-(CH₂)₂.

Die erfindungsgemäßen Verbindungen gemäß Formel (I) können in Gemischen mit Derivaten auftreten, die von der Formel (I) hinsichtlich des Alkylierungs-bzw. Protonierungsgrades sowie hinsichtlich des Carboxymethylierungsgrades abweichen, was z. B. herstellungsbedingt durch nicht vollständige Umsetzung der Fall sein kann.

Es können auch Mischungen der oben genannten Verbindungen hergestellt und verwendet werden.

Die amphiphilen grenzflächenaktiven Verbindungen gemäß dieser Erfindung eignen sich insbesondere als Emulgatoren, Demulgatoren, Detergenzien, Dispergatoren und Hydrotropica in Industrie und Haushalt, beispielsweise auf den Gebieten Metallbearbeitung, Erzgewinnung, Oberflächenveredelung, Waschen und Reinigen von Textilien oder harten Oberflächen, insbesondere als manuelles Spülmittel, sowie Waschen und Reinigen von Haut und Haar, Kosmetik, Medizin, Agrochemikalien und Nahrungsmittelverarbeitung und - zubereitung. Hierbei können sie mit allen gängigen anionischen, nichtionischen, kationischen und ampholytischen grenzflächenaktiven Substanzen kombiniert werden.

Ohne Formulierungen mit den erfindungsgemäßen Tensiden darauf einzuschränken seien für Kombinationen als Beispiele für nichtionische grenzflächenaktive Substanzen Fettsäureglyceride, Fettsäurepolyglyceride, Fettsäureester, Alkoxylate höherer Alkohole, alkoxylierte Fettsäureglyceride, Polyoxyethylenoxypropylenglykolfettsäureester, Polyoxyethylensorbitanfettsäureester, Polyoxyethylen-Rhizinusöl- oder gehärtete Rhizinusölderivate, Polyoxyethylenlanolinderivate, Polyoxyethylenfettsäureamide, Polyoxyethylenalkylamine, Derivate von Alkanolaminen, Alkylaminoxide, Derivate von Eiweißhydrolysaten, Hydroxymischether, Alkylpolyglycoside und Alkylglucamide (z. B. N-Methyl-alkylglucamide) sowie nichtionische Geminitenside, bzw. verbrückte nichtionische Tenside (wie in WO 95/19951 (Polyhydroxyaminverbindungen), WO 95/19953, WO 95/19954 und WO 95/19955 sowie WO 95/20026 beschrieben) genannt.

Als Beispiele für anionische grenzflächenaktive Substanzen, die für Kombinationen eingesetzt werden können, seien Seifen, Ethercarbonsäuren und deren Salze, Alkylsulfonate, α-Olefinsulfonate, α-Sulfofettsäurederivate (einschließlich der in WO 93/25646 beschriebenen), Sulfonate höherer Fettsäureester, höhere Alkoholsulfate (primär und sekundär), Alkoholethersulfate, Hydroxymischethersulfate, Sulfate von alkoxylierten Carbonsäurealkanolamiden, Salze von Phosphatestern, Tauride, Isethionate, lineare Alkylbenzolsulfonate, verbrückte Alkylbenzolsulfonate (wie DOWFAX-Typen der Firma Dow), Alkylarylsulfonate, Sulfate der Polyoxyethylenfettsäureamide und Derivate von Acylaminosäuren, Alkylethercarbonsäuren, Alkyl- und Dialkylsulfosuccinate, Alkenylsulfosuccinate, Alkyl- oder Alkenylsarcosinate und sulfatierte Glycerinalkylether und Geminitenside, wie sie in der deutschen Patentanmeldung 195 05 368.0 beschrieben werden, genannt.

Als Beispiele für kationische gängige grenzflächenaktive Substanzen, die für Kombinationen eingesetzt werden können, seien Alkyltrimethylammoniumsalze, Dialkyldimethylammoniumsalze, Alkyldimethylbenzylammoniumsalze, Alkylpyridiniumsalze, quaternierte Fettsäureester von Alkanolaminen, Alkylisochinoliniumsalze, Benzethoniumchloride und kationische Acylaminosäurederivate genannt.

Als Beispiele für Ampholyte und Betaine, die für Kombinationen eingesetzt werden können, seien Carbobetaine, wie z. B. Kokosacylamido-propyldimethylbetain, Acylamidopentandiethylbetain, Acylamidopropan-(oder -ethan-)dimethyl (oder-diethyl-)betain - alle mit C-Kettenlängen zwischen 10 und 18 , Sulfobetaine, Imidazolinderivate, Sojaöllipide und Lecithin genannt. Die oben erwähnten Amin-N-oxide können auch in polymerer Form vorliegen, wobei ein Verhältnis Amin- zu Amin-N-oxid von 10 : 1 bis 1 : 1 000 000 vorliegen muß. Die mittlere Molmasse beträgt 500 bis 1 000 000, besonders bevorzugt jedoch 5 000 bis 100 000.

Den erfindungsgemäßen grenzflächenaktiven Verbindungen können ebenfalls gängige Additive zugesetzt werden. Solche Additive werden speziell für eine Formulierung ausgewählt und umfassen üblicherweise anorganische Salze, wie Natriumchlorid und -sulfat, oder auch wasserlösliche Calcium- und / oder Magnesiumsalze sowie Builder, Hydrotropica, UV-Absorber, Weichmacher, Chelatbildner, Viskositätsmodifizierer, Enzyme, Schmutzlösepolymere, Bleichmittel, Bleichaktivatoren, Antiredepositionsadditive, polymere Dispergatoren, optische Aufheller, Additive zur Schaumregulierung und Riechstoffe.

Die obengenannten erfindungsgemäßen Verbindungen lassen sich nach bekannten Verfahren herstellen. Ohne die Herstellung darauf einzuschränken, soll hier die Umsetzung von Di-, Oligo- oder Polyaminen mit Aldehyden einer C-Kettenlänge von 6 bis 22 genannt werden. Hier bildet sich zuerst im einfachsten Falle ein Alkylendiimin, das dann im Folgeschritt mit Wasserstoff in Gegenwart eines Übergangsmetallkatalysators reduziert wird und im letzten Schritt mit Chloressigsäure oder ihrem Alkalisalz, insbesondere Natriumsalz, carboxymethyliert wird. Auf diese Weise kann man sicherstellen, daß die Tensideinheiten recht selektiv gebildet werden.

Die Carboxymethylierung wird bei einer Temperatur von 120 bis 160 °C, bevorzugt bei einer Temperatur von 115 bis 145 °C, durchgeführt. Die Reaktionszeit, die erforderlich ist, um den Gehalt an Mono- und Dichloressigsäure auf < 10 ppm zu senken, beträgt abhängig von der Reaktionstemperatur 1 bis 10 Stunden. Bevorzugt ist die Durchführung der Reaktion in zwei Schritten.

Die resultierenden Produkte zeichnen sich im Vergleich zu ihren konventionellen Äquivalenten durch signifikant niedrigere kritische Mizellbildungskonzentrationen sowie Oberflächenspannungen der wäßrigen Lösungen der erfindungsgemäßen Tenside als auch der Grenzflächenspannungen zwischen der besagten wäßrigen Lösung und verschiedenen Ölen, wie Paraffinöl aber auch Thymianöl oder verschiedene Triglyceride, aus. Darüber hinaus zeigen die erfindungsgemäßen Tenside eine außerordentliche Mildheit und Hautfreundlichkeit.

## Patentansprüche

1. Amphiphile amphotere Verbindungen der Formel (I) 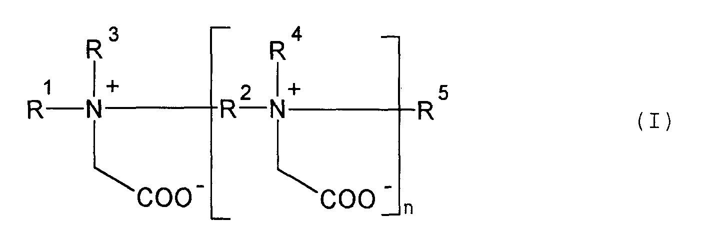 in der
R¹ für einen gesättigten oder ungesättigten, verzweigten oder unverzweigten, cyclischen oder acyclischen Kohlenwasserstoffrest mit 6 bis 22 Kohlenstoffatomen,
R² für, einen Spacer ausgewählt aus der Gruppe:
-(CH₂)₂-, -(CH₂)₃-, -(CH₂)₄-, -(CH₂)₅-, -(CH₂)₆-,
-CH₂-CH(CH₃)-(CH₂)₃-
- (CH₂)₂-O-(CH₂)₂-, -(CH₂)₂-O-(CH₂)₃-, -(CH₂)₃-O-(CH₂)₃-,
-(CH₂)₂-O-(CH₂)₂-O-(CH₂)₂-, -(CH₂)₃-O-(CH₂)₂-O-(CH₂)₃-,
-(CH₂)₂-O-(CH₂)₃-O-(CH₂)₂-,
- (CH₂)₂-NH-(CH₂)₃- und - (CH₂)₂-NH-(CH₂)₂ -,
R³ für ein Wasserstoffatom oder einen Alkylrest mit 1 bis 4 Kohlenstoffatomen,
R⁴ für einen gesättigten oder ungesättigten, verzweigten oder unverzweigten, cyclischen oder acyclischen Kohlenwasserstoffrest mit 6 bis 22 Kohlenstoffatomen,
R⁵ für ein Wasserstoffatom oder einen Alkylrest mit 1 bis 4 Kohlenstoffatomen und
n für eine Zahl von 1 bis 100000 steht, und
deren Mischungen.

2. Amphiphile amphotere Verbindungen nach Anspruch 1, dadurch gekennzeichnet, daß n in der Formel (I) einen Wert von 1 bis 1000 annimmt.

3. Verfahren zur Herstellung der amphiphilen amphoteren Verbindungen nach einem der vorhergehenden Ansprüche, dadurch gekennzeichnet, daß Di-, Oligo- oder Polyamine mit Aldehyden mit 6 bis 22 Kohlenstoffatomen umgesetzt werden und das Umsetzungsprodukt mit Wasserstoff reduziert und mit Chloressigsäure oder ihrem Natriumsalz carboxymethyliert wird.

4. Verwendung der amphiphilen Verbindungen nach einem der Ansprüche 1 bis 2 als Emulgatoren oder Demulgatoren.

5. Verwendung der amphiphilen Verbindungen nach einem der Ansprüche 1 bis 2 als Hilfsmittel bei der Metallbearbeitung, Erzgewinnung oder Oberflächenveredelung.

6. Verwendung der amphiphilen Verbindungen nach einem der Ansprüche 1 bis 2 für das Dispergieren von Agrochemikalien.

7. Verwendung der amphiphilen Verbindungen nach einem der Ansprüche 1 bis 2 für das Waschen und Reinigen von Textilien.

8. Verwendung der amphiphilen Verbindungen nach einem der Ansprüche 1 bis 2 für das Reinigen von harten Oberflächen, insbesondere als manuelles Spülmittel.

9. Verwendung der amphiphilen Verbindungen nach einem der Ansprüche 1 bis 2 für das Reinigen und Waschen von Haut und Haar.

## Claims

1. Amphiphilic amphoteric compounds of the formula (I) 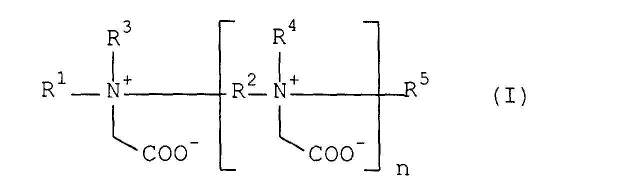 where
**R**^{**1**} is a saturated or unsaturated, branched or unbranched, cyclic or acyclic hydrocarbon radical having from 6 to 22 carbon atoms,
**R**^{**2**} is a spacer selected from the group consisting of
-(CH₂)₂-, -(CH₂)₃-, -(CH₂)₄-, -(CH₂)₅-,
-(CH₂)₆-, -CH₂-CH(CH₃)-(CH₂)₃-
-(CH₂)₂-O-(CH₂)₂-, -(CH₂)₂-O- (CH₂)₃-,
-(CH₂)₃-O-(CH₂)₃-, -(CH₂)₂-O-(CH₂)₂-O-(CH₂)₂-,
-(CH₂)₃-O-(CH₂)₂-O-(CH₂)₃-,
-(CH₂)₂-O-(CH₂)₃-O-(CH₂)₂-, -(CH₂)₂-NH-(CH₂)₃-,
and -(CH₂)₂-NH-(CH₂)₂-,
**R**^{**3**} is a hydrogen atom or an alkyl group having from 1 to 4 carbon atoms,
**R**^{**4**} is a saturated or unsaturated, branched or unbranched, cyclic or acyclic hydrocarbon radical having from 6 to 22 carbon atoms,
**R**^{**5**} is a hydrogen atom or an alkyl group having from 1 to 4 carbon atoms, and
**n** is a number from 1 to 100,000 and mixtures thereof.

2. The amphiphilic amphoteric compounds defined by claim 1,
**characterized in that** n in the formula (I) has a value from 1 to 1,000.

3. A process for producing the amphiphilic amphoteric compounds according to any one of the preceding claims,
**characterized in that** di-, oligo-, or polyamines are reacted with aldehydes having from 6 to 22 carbon atoms and the reaction product is reduced with hydrogen and carboxymethylated with chloroacetic acid or the sodium salt thereof.

4. Use of the amphiphilic compounds according to any one of claims 1 to 2 as emulsifiers or demulsifiers.

5. Use of the amphiphilic compounds according to any one of claims 1 to 2 as auxiliaries in metal working, ore mining, or surface finishing.

6. Use of the amphiphilic compounds according to any one of claims 1 to 2 for dispersing agrochemicals.

7. Use of the amphiphilic compounds according to any one of claims 1 to 2 for washing and cleaning textiles.

8. Use of the amphiphilic compounds according to any one of claims 1 to 2 for cleaning hard surfaces, particularly as a manual detergent.

9. Use of the amphiphilic compounds according to any one of claims 1 to 2 for cleaning and washing skin and hair.

## Revendications

1. Composés amphotères amphiphiles de formule (I) 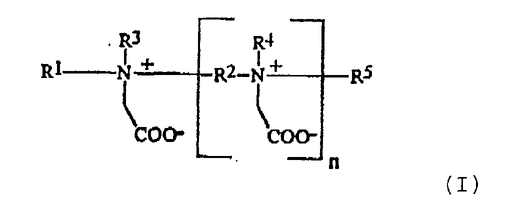 dans laquelle
R¹ représente un radical hydrocarboné saturé ou insaturé, ramifié ou non ramifié, cyclique ou acyclique, comprenant 6 à 22 atomes de carbone,
R2 représente un espaceur choisi parmi le groupe : - (CH₂)₂-, -(CH₂)₃-, -(CH₂)₄-, -(CH₂)₅-, -(CH₂)₆-, -CH₂-CH(CH₃)-(CH₂)₃-, -(CH₂)₂-O-(CH₂)₂-, -(CH₂)₂-O-(CH₂)₃-, -(CH₂)₃-O-(CH₂)₃-, - (CH₂)₂-O-(CH₂)₂-O-(CH₂)₂-, -(CH₂)₃-O-(CH₂)₂-O-(CH₂)₃-, - (CH₂)₂-O-(CH₂)₃-O-(CH₂)₂-, -(CH₂)₂-NH-(CH₂)₃- et -(CH₂)₂-NH-(CH₂)₂
R³ représente un atome d'hydrogène ou un radical alkyle comprenant 1 à 4 atomes de carbone,
R⁴ représente un radical hydrocarboné saturé ou insaturé, ramifié ou linéaire, cyclique ou acyclique comprenant 6 à 22 atomes de carbone,
R⁵ représente un atome d'hydrogène ou un radical alkyle comprenant 1 à 4 atomes de carbone
n représente un nombre de 1 à 100000, et leurs mélanges.

2. Composés amphotères amphiphiles selon la revendication 1, caractérisés en ce que n dans la formule (I) prend une valeur de 1 à 1000.

3. Procédé pour la préparation de composés amphotères amphiphiles selon l'une quelconque des revendications précédentes, caractérisé en ce qu'on transforme des diamines, des oligoamines ou des polyamines avec des aldéhydes comprenant 6 à 22 atomes de carbone et en ce qu'on réduit le produit de la transformation avec de l'hydrogène et en ce qu'on réalise une carboxyméthylation avec de l'acide chloroacétique ou son sel sodique.

4. Utilisation des composés amphiphiles selon l'une quelconque des revendications 1 à 2 comme émulsifiants ou désémulsifiants.

5. Utilisation des composés amphiphiles selon l'une quelconque des revendications 1 à 2 comme adjuvants lors du traitement de métaux, de l'extraction de minerais ou du finissage de surfaces.

6. Utilisation des composés amphiphiles selon l'une quelconque des revendications 1 à 2 pour la dispersion de produits agrochimiques.

7. Utilisation des composés amphiphiles selon l'une quelconque des revendications 1 à 2 pour le lavage et le nettoyage de textiles.

8. Utilisation des composés amphiphiles selon l'une quelconque des revendications 1 à 2 pour le nettoyage de surfaces dures, en particulier comme agent de rinçage manuel.

9. Utilisation des composés amphiphiles selon l'une quelconque des revendications 1 à 2 pour le nettoyage et le lavage de la peau et des cheveux.
